# EUROPEAN PATENT APPLICATION

(11) **EP 4 692 031 A1**
(43) Date of publication of application: **11.02.2026**
(21) Application number: 24780450.3
(22) Date of filing: 27.03.2024
(51) Int. Cl.: C07C 1/20, C07C 15/08, C07C 29/151, C07C 31/04

(54) **SYSTEM AND METHOD FOR PRODUCING AROMATIC HYDROCARBON**

(30) Priority: 31.03.2023 JP 2023058843
(71) Applicant: KAWASAKI JUKOGYO KABUSHIKI KAISHA, Kobe-shi, Hyogo 650-8670 (JP)
(72) Inventor: YONEDA, Koji, Hyogo, 650-8670 (JP); YAMADA, Ryuhei, Hyogo, 650-8670 (JP); WARATANI, Yusuke, Hyogo, 650-8670 (JP); TANIYAMA, Noriyuki, Hyogo, 650-8670 (JP); KIYOTAKI, Gen, Hyogo, 650-8670 (JP)
(74) Representative: Dehns
(86) International application number: PCT/JP2024/012250
(87) International publication number: WO 2024/204346

(57) **Abstract**

A system for producing aromatic hydrocarbons includes: a xylene synthesis reactor that is filled with a xylene synthesis catalyst and that synthesizes aromatic hydrocarbons from methanol by a xylene synthesis reaction in the presence of water vapor; a methanol feed line through which the methanol is fed to the xylene synthesis reactor; and a water vapor feed line through which the water vapor is fed to the xylene synthesis reactor.

## Description

### Technical Field

The present disclosure relates to a system for, and a method of, producing xylene-containing aromatic hydrocarbons by using CO₂ as a raw material.

### Background Art

Para-xylene is an aromatic hydrocarbon, and is an important basic chemical used as a raw material for purified terephthalic acid, which is used as a raw material for polyester fibers or PET bottle resin. Conventionally, para-xylene derived from fossil fuel has been produced. In recent years, it has been proposed to industrially produce para-xylene by using CO₂ as a raw material. This approach is one of the "carbon recycling technologies" aiming to capture and recover CO₂ emitted from a factory or the like and efficiently utilize the recovered CO₂ by treating it as a resource.

A process of industrially producing para-xylene by using CO₂ as a raw material includes: (1) a methanol synthesis step of synthesizing methanol from CO₂ and H₂: (2) a xylene synthesis step of synthesizing xylenes from the methanol; and (3) a para-xylene separation step of separating para-xylene from the xylenes. There are three types of xylene isomers: para-xylene, ortho-xylene, and meta-xylene. In the para-xylene separation step, para-xylene is selectively separated from xylene-containing aromatic hydrocarbons.

There is a known conventional technique in which methanol is brought into contact with a zeolite-based catalyst to convert the methanol into aromatic hydrocarbons. For example, Patent Literature 1 discloses a technique to synthesize xylenes from methanol by using a zeolite-based catalyst.

### Citation List

### Patent Literature

PTL 1: Japanese Laid-Open Patent Application Publication No. 2007-137840

### Summary of Invention

### Technical Problem

In a reaction to synthesize xylenes from methanol by using a xylene synthesis catalyst, if the xylene synthesis catalyst becomes deactivated, the xylene synthesis reaction stops. The longer the reaction time before the xylene synthesis catalyst becomes deactivated, i.e., the longer the service life of the catalyst, the lower the frequency and cost of the maintenance, such as replacement of the catalyst.

The present disclosure has been made in view of the above, and an object of the present disclosure is to provide a technique that, in a system for and a method of producing aromatic hydrocarbons, makes it possible to extend the reaction time before the deactivation of a xylene synthesis catalyst that promotes the synthesis of xylenes from methanol, i.e., to extend the service life of the catalyst.

### Solution to Problem

The inventors of the present invention have conducted diligent studies, and consequently have newly found that, in a reaction to synthesize xylenes from methanol by using a xylene synthesis catalyst, the time before the deactivation of the xylene synthesis catalyst, i.e., the service life of the catalyst, is extended by adding water vapor to the methanol.

A system for producing aromatic hydrocarbons according to one aspect of the present disclosure includes: a xylene synthesis reactor that is filled with a xylene synthesis catalyst and that synthesizes aromatic hydrocarbons from methanol and water vapor by a xylene synthesis reaction; a methanol feed line through which the methanol is fed to the xylene synthesis reactor; and a water vapor feed line through which the water vapor is fed to the xylene synthesis reactor.

A method of producing aromatic hydrocarbons according to one aspect of the present disclosure includes: feeding methanol to a xylene synthesis reactor filled with a xylene synthesis catalyst; feeding water vapor to the xylene synthesis reactor; and synthesizing, by the xylene synthesis reactor, aromatic hydrocarbons from the methanol and the water vapor by a xylene synthesis reaction.

### Advantageous Effects of Invention

The present disclosure makes it possible to provide a technique that, in a system for and a method of producing aromatic hydrocarbons, makes it possible to extend the reaction time before the deactivation of a xylene synthesis catalyst that promotes the synthesis of xylenes from methanol, i.e., to extend the service life of the catalyst.

### Brief Description of Drawings

FIG. 1 is a chart showing a relationship between the reaction time of a xylene synthesis catalyst and a methanol conversion rate when the water vapor concentration in a raw material is varied in a xylene synthesis reaction using the xylene synthesis catalyst.
FIG. 2 is a block diagram showing an overall configuration of an aromatic hydrocarbon production system according to one embodiment of the present disclosure.
FIG. 3 is a block diagram showing an overall configuration of the aromatic hydrocarbon production system according to Variation 1.

### Description of Embodiments

In a system for producing aromatic hydrocarbons, i.e., an aromatic hydrocarbon production system, according to the present disclosure, methanol is brought into contact with a xylene synthesis catalyst to utilize a reaction to convert the methanol into xylene-containing aromatic hydrocarbons (this reaction is hereinafter referred to as "xylene synthesis reaction"). In the aromatic hydrocarbon production system according to the present disclosure, by bringing methanol into contact with the xylene synthesis catalyst in the presence of water vapor, aromatic hydrocarbons are generated while reducing the deactivation rate of the catalyst.

A test was conducted on a xylene synthesis reaction using a xylene synthesis catalyst to confirm a relationship between the water vapor concentration in a raw material and the service life of the xylene synthesis catalyst. In the test, a zeolite-based catalyst was used as the xylene synthesis catalyst, and the raw material having a temperature of 400°C was prepared, in which 16 mol% of methanol, α mol% of water vapor, and (84-α) mol% of nitrogen were mixed together. The raw material having a temperature of 400°C was continuously fed to a reaction vessel filled with the xylene synthesis catalyst, and the amount of methanol contained in exhaust gas from the reaction vessel was measured every hour. From the measurement values, a methanol conversion rate [%] was determined. The methanol conversion rate indicates the proportion of the amount of methanol that has converted into aromatic hydrocarbons to the total amount of methanol fed, the proportion being expressed as a percentage. The water vapor concentration α was used as a variable, and was varied among 0, 4, 8, and 16.

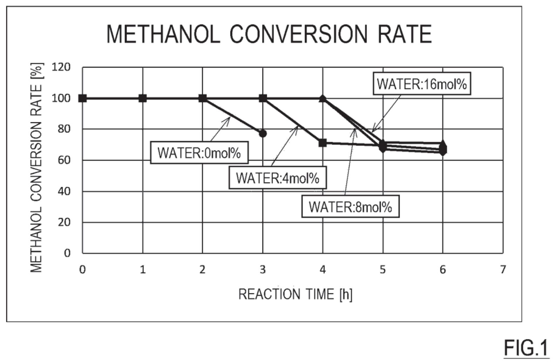

FIG. 1 is a chart showing a relationship between the reaction time of the xylene synthesis catalyst and the methanol conversion rate when the water vapor concentration in the raw material is varied in the xylene synthesis reaction using the xylene synthesis catalyst. In the chart of FIG. 1, the vertical axis represents the methanol conversion rate [%], and the horizontal axis represents the reaction time of the xylene synthesis catalyst. In a case where the water vapor concentration in the raw material is 0 mol%, the methanol conversion rate during a period from the start of the reaction until the elapse of 2 hours is 100%, but after the elapse of 2 hours, the methanol conversion rate drops to about 80%. In a case where the water vapor concentration in the raw material is 4 mol%, the methanol conversion rate from the start of the reaction until the elapse of 3 hours is 100%, but after the elapse of 4 hours, the methanol conversion rate drops to about 70%. In a case where the water vapor concentration in the raw material is 8 mol%, the methanol conversion rate from the start of the reaction until the elapse of 4 hours is 100%, but after the elapse of 5 hours, the methanol conversion rate drops to about 70%. In a case where the water vapor concentration in the raw material is 16 mol%, the methanol conversion rate from the start of the reaction until the elapse of 4 hours is 100%, but after the elapse of 5 hours, the methanol conversion rate drops to about 70%. From these results, it can be concluded as follows: in the case where the water vapor concentration in the raw material is 0 mol%, the xylene synthesis catalyst becomes deactivated in 3 hours; in the case where the water vapor concentration in the mixture gas is 4 mol%, the xylene synthesis catalyst becomes deactivated in 4 hours; and in the cases where the water vapor concentration in the mixture gas is 8 mol% and 16 mol%, respectively, the xylene synthesis catalyst becomes deactivated in 5 hours. Thus, it has been found that by bringing methanol into contact with the xylene synthesis catalyst in the presence of water vapor, aromatic hydrocarbons can be generated while reducing the deactivation rate of the catalyst. The service life of the catalyst is extended as a result of the reduction of the deactivation rate of the catalyst. Further, it has been indicated that in a case where the methanol concentration in the raw material brought into contact with the xylene synthesis catalyst is 16 mol%, the catalyst deactivation rate reduction effect can be obtained if the water vapor concentration in the raw material is 4 mol% or greater, i.e., if the ratio of the water vapor concentration to the methanol concentration is 1/4 or greater. It has also been indicated that in the case where the methanol concentration in the raw material brought into contact with the xylene synthesis catalyst is 16 mol%, the catalyst deactivation rate reduction effect does not change if the water vapor concentration in the raw material is 8 mol% or greater, i.e., if the ratio of the water vapor concentration to the methanol concentration is 1/2 or greater.

### <<Configuration of Aromatic Hydrocarbon Production System 100>>

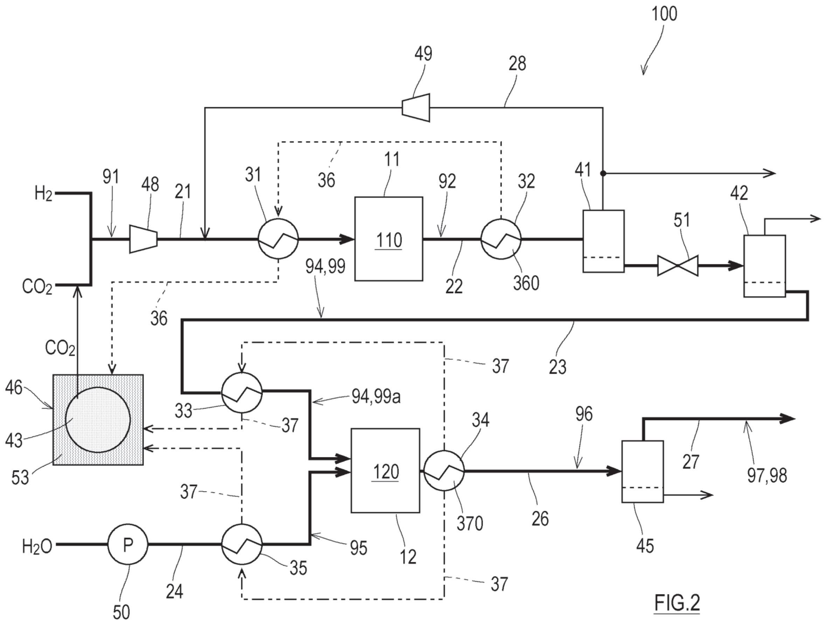

FIG. 2 is a block diagram showing an overall configuration of an aromatic hydrocarbon production system 100 according to the present disclosure. In a process of industrially producing para-xylene by using H₂ (hydrogen) and CO₂ (carbon dioxide) as raw materials, the aromatic hydrocarbon production system 100 according to the present disclosure performs (1) a methanol synthesis step of synthesizing methanol from CO₂ and H₂ and (2) a xylene synthesis step of synthesizing xylenes from the methanol, thereby producing xylene-containing aromatic hydrocarbons 97.

The aromatic hydrocarbon production system 100 includes a methanol synthesis reactor 11 and a xylene synthesis reactor 12.

The methanol synthesis reactor 11 is a reaction vessel in which methanol is synthesized by hydrogenation of CO₂. The methanol synthesis reactor 11 is filled with a methanol synthesis catalyst 110. The methanol synthesis catalyst 110 catalyzes a methanol synthesis reaction to generate methanol and water vapor from CO₂ and H₂ (CO₂ + 3H₂ → CH₃OH + H₂O). The methanol synthesis catalyst 110 is not particularly limited, so long as it catalyzes a reaction to generate methanol from CO₂. Known examples of the methanol synthesis catalyst 110 include a Cu-based catalyst (for a reaction temperature of about 250°C and a reaction pressure of 3 to 5 MPa) and a metal oxide catalyst.

A raw material gas line 21 and a first reaction product line 22 are connected to the methanol synthesis reactor 11.

Through the raw material gas line 21, raw material gas 91 containing H₂ and CO₂ is fed to the methanol synthesis reactor 11. H₂ in the raw material gas 91 is, for example, generated by electrolysis of water. CO₂ in the raw material gas 91 is fed from a carbon dioxide feed source 46. The carbon dioxide feed source 46 will be described below in detail.

The raw material gas line 21 includes a compressor 48. The compressor 48 increases the pressure of the raw material gas 91 to a suitable pressure for the methanol synthesis reaction. The raw material gas line 21 further includes a raw material heater 31. The raw material heater 31 increases the temperature of the raw material gas 91 to a suitable temperature for the methanol synthesis reaction. The reaction temperature and reaction pressure of the methanol synthesis reaction differ depending on the methanol synthesis catalyst 110. For example, in a case where the methanol synthesis catalyst 110 is a Cu-based catalyst, the reaction temperature is about 250°C and the reaction pressure is 3 to 5 MPa.

A first fluid 92 discharged from the methanol synthesis reactor 11 flows into the first reaction product line 22. The first fluid 92 flowing into the first reaction product line 22 contains methanol and water vapor, which are the reaction products of the methanol synthesis reaction, and also contains unreacted H₂ and CO₂.

The first reaction product line 22 includes first heat recovery equipment 32. The first heat recovery equipment 32 recovers the thermal energy of the first fluid 92. For example, the first heat recovery equipment 32 includes a first heat transfer medium passage 360, through which a first heat transfer medium 36 flows. The first heat transfer medium 36 recovers the thermal energy of the first fluid 92 by exchanging heat with the first fluid 92. In FIG. 2, chain lines indicate movement of the first heat transfer medium 36. The passage for the first heat transfer medium 36 is formed by piping or the like. The first heat transfer medium 36 that has recovered the thermal energy of the first fluid 92 exchanges heat with the raw material gas 91 at the raw material heater 31. That is, the thermal energy recovered from the first fluid 92 at the first heat recovery equipment 32 is utilized to heat the raw material gas 91.

A first gas-liquid separator 41 is located on the first reaction product line 22 at a position downstream of the first heat recovery equipment 32. The first gas-liquid separator 41 separates and removes gas components such as water vapor from the first fluid 92 flowing through the first reaction product line 22. The gas components separated from the first fluid 92 by the first gas-liquid separator 41 contain unreacted H₂ and CO₂. Part of the gas components separated from the first fluid 92 are returned to the raw material gas line 21 through a recycle line 28. The recycle line 28 includes a compressor 49. The pressure of the gas components that are returned from the first gas-liquid separator 41 to the raw material gas 91 is increased by the compressor 49 to a suitable pressure for the methanol synthesis reaction.

In the first reaction product line 22, downstream of the first gas-liquid separator 41, the first fluid 92 from which the gas components have been separated, i.e., a methanol-rich liquid, flows. This liquid also contains water that is generated as a result of liquefaction of the water vapor contained in the first fluid 92. A decompressor 51 is located on the first reaction product line 22 at a position downstream of the first gas-liquid separator 41. The decompressor 51 decreases the pressure of the first fluid 92.

A second gas-liquid separator 42 is located on the first reaction product line 22 at a position downstream of the decompressor 51. The components of the first fluid 92 that has vaporized as a result of the pressure decrease by the decompressor 51 are partly removed from the first fluid 92 by the second gas-liquid separator 42. In this manner, gas components and liquid components are separated from the first fluid 92 by the first gas-liquid separator 41 and the second gas-liquid separator 42, i.e., by two-stage gas-liquid separators, and thereby methanol 94 is separated from the first fluid 92. In the methanol 94, water generated as a result of liquefaction of water vapor is being mixed. A methanol feed line 23 is connected to the second gas-liquid separator 42. The methanol 94 is fed to the xylene synthesis reactor 12 through the methanol feed line 23. The fluid flowing through the methanol feed line 23 may contain water 99 (or water vapor 99a) in addition to the methanol 94.

The methanol feed line 23, a water vapor feed line 24, and a second reaction product line 26 are connected to the xylene synthesis reactor 12.

The xylene synthesis reactor 12 is a reaction vessel to perform the xylene synthesis reaction. The xylene synthesis reactor 12 is filled with a xylene synthesis catalyst 120. The xylene synthesis catalyst 120 catalyzes a synthesis reaction to convert methanol into aromatic hydrocarbons in the presence of water vapor. The xylene synthesis catalyst 120 is not particularly limited to a specific type. As the xylene synthesis catalyst 120, for example, a zeolite-based catalyst is known, and the reaction temperature of the xylene synthesis catalyst 120 is about 400 to 600°C.

The methanol 94 is fed to the xylene synthesis reactor 12 through the methanol feed line 23. The methanol feed line 23 includes a methanol heater 33. The methanol heater 33 increases the temperature of the methanol 94 to a suitable temperature for the xylene synthesis reaction. Through the methanol feed line 23, vaporized methanol 94 and water 99 may be fed to the xylene synthesis reactor 12. In this case, the methanol feed line 23 doubles as a water vapor feed line. The water 99 and the methanol 94 that have flowed out of the second gas-liquid separator 42 into the methanol feed line 23 are heated by the methanol heater 33 located on the methanol feed line 23 to turn into water vapor 99a and gaseous methanol 94, and then flow into the xylene synthesis reactor 12.

Water vapor 95 is fed to the xylene synthesis reactor 12 through the water vapor feed line 24. The water vapor 95 may be diluted with nitrogen to adjust the water vapor concentration in the xylene synthesis reactor 12. The water vapor feed line 24 includes a pump 50. The pump 50 increases the pressure of the water vapor 95 to a suitable pressure for the xylene synthesis reaction, and then feeds the water vapor 95 to the xylene synthesis reactor 12. A water vapor heater 35 is located on the water vapor feed line 24 at a position downstream of the pump 50. The water vapor heater 35 increases the temperature of the water vapor 95 to a suitable temperature for the xylene synthesis reaction. In a case where the methanol feed line 23 doubles as a water vapor feed line as mentioned above, the water vapor feed line 24 may be eliminated.

When the methanol 94 and the water vapor 95 and 99a are fed to the xylene synthesis reactor 12, the xylene synthesis reaction is caused by the action of the xylene synthesis catalyst 120. Reaction products generated in the xylene synthesis reactor 12 contain aromatic hydrocarbons, and also contain light hydrocarbons. The aromatic hydrocarbons in the reaction products may contain benzene, toluene, and three types of xylene isomers. The light hydrocarbons in the reaction products may contain C₂ to C₄ alkenes, such as ethylene and propylene, and C₁ to C₅ alkanes, such as methane, ethane, propane, and butane. However, the composition and proportion of the aromatic hydrocarbons and light hydrocarbons contained in the reaction products vary depending on the type of the xylene synthesis catalyst 120.

A second fluid 96 discharged from the xylene synthesis reactor 12 flows into the second reaction product line 26. The second fluid 96 flowing into the second reaction product line 26 contains the aromatic hydrocarbons that are the reaction products of the xylene synthesis reaction, and also contains light hydrocarbons and water vapor.

The second reaction product line 26 includes second heat recovery equipment 34. The second heat recovery equipment 34 recovers the thermal energy of the second fluid 96. For example, the second heat recovery equipment 34 includes a second heat transfer medium passage 370, through which a second heat transfer medium 37 flows. The second heat transfer medium 37 recovers the thermal energy of the second fluid 96 by exchanging heat with the second fluid 96. In FIG. 2, two-dot chain lines indicate movement of the second heat transfer medium 37. The passage for the second heat transfer medium 37 is formed by piping or the like. The second heat transfer medium 37 that has recovered the thermal energy of the second fluid 96 exchanges heat with the methanol 94 at the methanol heater 33. That is, the thermal energy recovered from the second fluid 96 at the second heat recovery equipment 34 is utilized to heat the methanol 94. Also, the second heat transfer medium 37 that has recovered the thermal energy from the second fluid 96 exchanges heat with the water vapor 95 at the water vapor heater 35. That is, the thermal energy recovered from the second fluid 96 at the second heat recovery equipment 34 is utilized to heat the water vapor 95.

A third gas-liquid separator 45 is located on the second reaction product line 26 at a position downstream of the second heat recovery equipment 34. The third gas-liquid separator 45 separates the second fluid 96 into gas components and liquid components. The liquid components of the second fluid 96 contain liquefied water vapor, i.e., water, and the xylene-containing aromatic hydrocarbons 97. The water and the aromatic hydrocarbons 97 are separated based on their specific gravities. The gas components of the second fluid 96 contain aliphatic hydrocarbons 98 containing olefin-based light hydrocarbons such as ethylene and propylene.

A product line 27 is connected to the third gas-liquid separator 45. The aromatic hydrocarbons 97 separated from the second fluid 96 by the third gas-liquid separator 45 flow into the product line 27. The aromatic hydrocarbons 97 are fed to the next para-xylene separation step through the product line 27. In the para-xylene separation step, para-xylene is separated from the aromatic hydrocarbons 97, and the separated para-xylene is used as a material for chemical products.

### <<Configuration of Carbon Dioxide Feed Source 46>>

The carbon dioxide feed source 46 of the aromatic hydrocarbon production system 100 according to the present disclosure includes: a carbon dioxide absorbent 43, which reversibly absorbs CO₂ by chemical absorption; and an absorbent heater 53, which heats the carbon dioxide absorbent 43. In the carbon dioxide feed source 46, the carbon dioxide absorbent 43 is heated by the absorbent heater 53, and as a result, the carbon dioxide absorbent 43 desorbs CO₂ stored therein. The desorbed CO₂ is fed to the raw material gas line 21 to be used as the raw material gas 91. The carbon dioxide absorbent 43 is, after desorbing CO₂ stored therein, used for absorbing CO₂ again.

As the carbon dioxide absorbent 43, an amine-based carbon dioxide absorbent 43 in liquid form (i.e., a carbon dioxide chemical absorption solution) is used. Alternatively, as the carbon dioxide absorbent 43, a carbon dioxide absorbent 43 in solid form (i.e., a carbon dioxide chemical absorbent material) may be used. The carbon dioxide absorbent 43 in solid form includes a porous support, such as zeolite, on which an amine compound that reversibly absorbs CO₂ is supported. Examples of amines typically used in the carbon dioxide absorbent 43 include: alkanolamines such as monoethanolamine and methyldiethanolamine; sterically hindered amines such as 2-amino-2-methyl-1-propanol; and cyclic amines such as piperazine. The CO₂ absorption temperature and CO₂ desorption temperature of the carbon dioxide absorbent 43 very depending on, for example, the properties of the carbon dioxide absorbent 43 and the amine type. For example, in a case where the carbon dioxide absorbent 43 utilizes a neutralization reaction between an aqueous alkanolamine solution and CO₂, the carbon dioxide absorbent 43 absorbs CO₂ at 40 to 50°C and desorbs CO₂ at 110 to 130°C. For the CO₂ desorption from the carbon dioxide absorbent 43, a suitable technique is adopted in accordance with the type of the carbon dioxide absorbent 43. As one example, by indirectly heating the carbon dioxide absorbent 43 in liquid form, CO₂ can be desorbed from the carbon dioxide absorbent 43. As another example, by indirectly heating the carbon dioxide absorbent 43 in solid form or by bringing the carbon dioxide absorbent 43 into contact with water vapor, CO₂ can be desorbed from the carbon dioxide absorbent 43.

The configuration of the carbon dioxide feed source 46 is not particularly limited. For example, the carbon dioxide feed source 46 may configured as described below.
(i) In the case of adopting the carbon dioxide absorbent 43 in liquid form, the carbon dioxide feed source 46 includes: an absorption vessel and a desorption vessel, each of which accommodates the carbon dioxide absorbent 43; and the absorbent heater 53, which indirectly heats the carbon dioxide absorbent 43 in the desorption vessel. The absorption vessel and the desorption vessel are in communication with each other. Gas containing CO₂ is fed to the absorption vessel, and the carbon dioxide absorbent 43 therein absorbs CO₂. After absorbing CO₂, the carbon dioxide absorbent 43 moves to the desorption vessel. In the desorption vessel, the carbon dioxide absorbent 43 is heated, and consequently the carbon dioxide absorbent 43 desorbs CO₂. After desorbing CO₂, the carbon dioxide absorbent 43 moves to the absorption vessel.
(ii) In the case of adopting the carbon dioxide absorbent 43 in solid form, the carbon dioxide feed source 46 includes a series of reaction vessels through which the carbon dioxide absorbent 43 flows. While part of the carbon dioxide absorbent 43 is being used for CO₂ absorption, part of the remaining carbon dioxide absorbent 43 desorbs CO₂ as a result of being heated by the absorbent heater 53.
(iii) In the case of adopting the carbon dioxide absorbent 43 in solid form, the carbon dioxide feed source 46 includes multiple reaction vessels filled with the carbon dioxide absorbent 43. While part of the reaction vessels is being used for CO₂ absorption, part of the remaining reaction vessels desorbs CO₂ as a result of being heated by the absorbent heater 53.

For the CO₂ desorption from the carbon dioxide absorbent 43, the thermal energy recovered from the first fluid 92, i.e., part of the heat generated from the methanol synthesis, may be utilized. In this case, the first heat transfer medium 36 that has recovered the thermal energy of the first fluid 92 at the first heat recovery equipment 32 flows through the raw material heater 31, at which the first heat transfer medium 36 exchanges heat with the raw material gas 91 to heat the raw material gas 91. The first heat transfer medium 36 is then fed to the absorbent heater 53 of the carbon dioxide feed source 46, at which the first heat transfer medium 36 is used to heat the carbon dioxide absorbent 43.

Further, for the CO₂ desorption from the carbon dioxide absorbent 43, the thermal energy recovered from the second fluid 96, i.e., part of the heat generated from the xylene synthesis reaction, may be utilized. In this case, the second heat transfer medium 37 that has recovered the thermal energy of the second fluid 96 at the second heat recovery equipment 34 flows through the methanol heater 33, at which the second heat transfer medium 37 exchanges heat with the methanol 94 to heat the methanol 94. The second heat transfer medium 37 is then fed to the absorbent heater 53 of the carbon dioxide feed source 46, at which the second heat transfer medium 37 is used to heat the carbon dioxide absorbent 43. Also, the second heat transfer medium 37 that has recovered the thermal energy of the second fluid 96 at the second heat recovery equipment 34 flows through the water vapor heater 35, at which the second heat transfer medium 37 exchanges heat with the water vapor 95 to heat the water vapor 95. The second heat transfer medium 37 is then fed to the absorbent heater 53 of the carbon dioxide feed source 46, at which the second heat transfer medium 37 is used to heat the carbon dioxide absorbent 43.

At the carbon dioxide feed source 46, to desorb CO₂ from the carbon dioxide absorbent 43, at least one of the thermal energy recovered from the first fluid 92 or the thermal energy recovered from the second fluid 96 may be used. Here, in order to achieve more effective utilization of the thermal energy within the system, it is preferable that both the thermal energy recovered from the first fluid 92 and the thermal energy recovered from the second fluid 96 be utilized to desorb CO₂ from the carbon dioxide absorbent 43.

The CO₂ desorption temperature of the carbon dioxide absorbent 43 is lower than the methanol synthesis reaction temperature, and the methanol synthesis reaction temperature is lower than the xylene synthesis reaction temperature. The methanol synthesis reaction and the xylene synthesis reaction are both exothermic reactions, and the temperature of the second fluid 96 discharged from the xylene synthesis reactor 12 is higher than the temperature of the first fluid 92 discharged from the methanol synthesis reactor 11. In light of such a temperature relationship within the system, in order to achieve further effective utilization of the thermal energy within the system, the thermal energy of the second fluid 96 recovered by the second heat recovery equipment 34 may be utilized to heat the raw material gas 91 and the methanol 94 and thereafter utilized to heat the carbon dioxide absorbent 43. Similarly, the thermal energy of the second fluid 96 recovered by the second heat recovery equipment 34 may be utilized to heat the raw material gas 91 and the water vapor 95 and thereafter utilized to heat the carbon dioxide absorbent 43.

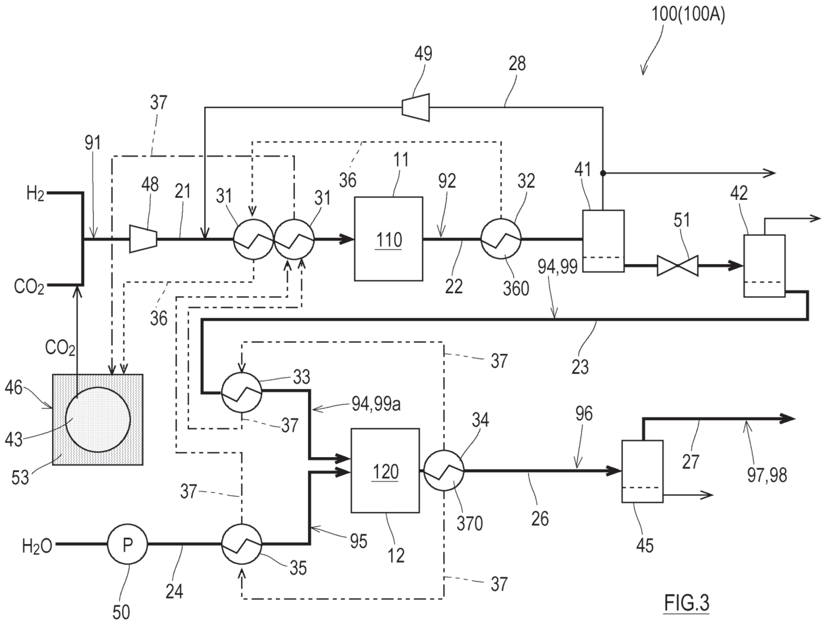

In the aromatic hydrocarbon production system 100 (100A) according to Variation 1 shown in FIG. 3, the raw material gas line 21 includes: a first raw material heater 31, which causes the first heat transfer medium 36 and the raw material gas 91 to exchange heat with each other; and a second raw material heater 31, which causes the second heat transfer medium 37 and the raw material gas 91 to exchange heat with each other. The first heat transfer medium 36 that has recovered the thermal energy of the first fluid 92 at the first heat recovery equipment 32 flows through the first raw material heater 31, at which the first heat transfer medium 36 exchanges heat with the raw material gas 91 to heat the raw material gas 91. The first heat transfer medium 36 is then fed to the absorbent heater 53, at which the first heat transfer medium 36 is used to heat the carbon dioxide absorbent 43. The second heat transfer medium 37 that has recovered the thermal energy of the second fluid 96 at the second heat recovery equipment 34 first flows through the methanol heater 33, at which the second heat transfer medium 37 exchanges heat with the methanol 94 to heat the methanol 94, then flows through the second raw material heater 31, at which the second heat transfer medium 37 exchanges heat with the raw material gas 91 to heat the raw material gas 91, and is then finally fed to the absorbent heater 53 of the carbon dioxide feed source 46, at which the second heat transfer medium 37 is used to heat the carbon dioxide absorbent 43. Also, the second heat transfer medium 37 that has recovered the thermal energy of the second fluid 96 at the second heat recovery equipment 34 first flows through the water vapor heater 35, at which the second heat transfer medium 37 exchanges heat with the water vapor 95 to heat the water vapor 95, then flows through the second raw material heater 31, at which the second heat transfer medium 37 exchanges heat with the raw material gas 91 to heat the raw material gas 91, and is then finally fed to the absorbent heater 53 of the carbon dioxide feed source 46, at which the second heat transfer medium 37 is used to heat the carbon dioxide absorbent 43. The aromatic hydrocarbon production system 100A according to Variation 1 is different from the aromatic hydrocarbon production system 100 according to the above-described embodiment in terms of the flow of the second heat transfer medium 37. Except this point, the aromatic hydrocarbon production system 100A according to Variation 1 is substantially the same in configuration as the aromatic hydrocarbon production system 100 according to the above-described embodiment.

### [Summary]

A system 100 for producing aromatic hydrocarbons 97 according to item 1 of the present disclosure includes: a xylene synthesis reactor 12, which is filled with a xylene synthesis catalyst 120 and which synthesizes aromatic hydrocarbons 97 from methanol 94 by a xylene synthesis reaction in the presence of water vapor 95, 99a; a methanol feed line 23, through which the methanol 94 is fed to the xylene synthesis reactor 12; and a water vapor feed line 23, 24, through which the water vapor 99a, 95 is fed to the xylene synthesis reactor 12.

In the xylene synthesis reactor 12, the concentration of the water vapor 99a, 95 in the gas that comes into contact with the xylene synthesis catalyst 120 is preferably 1/4 or greater of the concentration of the methanol 94 in the gas. The concentration ratio between the methanol 94 and the water vapor 99a, 95 in the gas that comes into contact with the xylene synthesis catalyst 120 may be about 1:1. Further, water may be added as a diluent to suppress the generation of heat during the reaction. In this case, the concentration of the water vapor 99a, 95 in the gas that comes into contact with the xylene synthesis catalyst 120 is preferably greater than the concentration of the methanol 94 in the gas. For example, the volume ratio between the water vapor 99a, 95 and the methanol 94 in the gas that comes into contact with the xylene synthesis catalyst 120 may be about 9:1. In an extreme example, the volume ratio between the water vapor 99a, 95 and the methanol 94 in the gas that comes into contact with the xylene synthesis catalyst 120 may be about 99:1.

In the xylene synthesis reactor 12 of the production system 100 configured as described above, the methanol 94 is converted into the aromatic hydrocarbons 97 by the xylene synthesis catalyst 120 in the presence of the water vapor 99a, 95. It has been found that by bringing the methanol 94 into contact with the xylene synthesis catalyst 120 in the presence of the water vapor 99a, 95, the aromatic hydrocarbons 97 can be generated while reducing the deactivation rate of the catalyst 120. Thus, in the aromatic hydrocarbon production system 100, the deactivation rate of the xylene synthesis catalyst 120 is reduced, which makes it possible to extend the service life of the xylene synthesis catalyst 120, and consequently the frequency of the replacement of the xylene synthesis catalyst 120 can be reduced.

The system 100 for producing aromatic hydrocarbons 97 according to item 2 of the present disclosure is configured such that the system 100 for producing aromatic hydrocarbons 97 according to item 1 includes: a methanol synthesis reactor 11, which synthesizes the methanol 94 from raw material gas 91 containing hydrogen and carbon dioxide by a methanol synthesis reaction accompanied by water vapor generation; a raw material gas line 21, through which the raw material gas 91 is fed to the methanol synthesis reactor 11; and a gas-liquid separator 41, 42, which separates the methanol 94 and water 99 from a first fluid 92, which contains reaction products of the methanol synthesis reaction and which has been discharged from the methanol synthesis reactor 11, wherein: through the methanol feed line 23, the methanol 94 and the water 99 separated from the first fluid 92 by the gas-liquid separator 44 are fed to the xylene synthesis reactor 12 as the methanol 94 and the water vapor 99a; and the methanol feed line 23 doubles as the water vapor feed line.

In the production system 100 configured as described above, water generated from the methanol synthesis reaction can be effectively utilized for the xylene synthesis reaction.

The system 100 for producing aromatic hydrocarbons 97 according to item 3 of the present disclosure is configured such that the system 100 for producing aromatic hydrocarbons 97 according to item 1 includes: a methanol synthesis reactor 11, which synthesizes the methanol 94 from raw material gas 91 containing hydrogen and carbon dioxide by a methanol synthesis reaction; a raw material gas line 21, through which the raw material gas 91 is fed to the methanol synthesis reactor 11; a gas-liquid separator 41, 42, which separates the methanol 94 from a first fluid 92, which contains reaction products of the methanol synthesis reaction and which has been discharged from the methanol synthesis reactor 11; and the methanol feed line 23, through which the methanol 94 separated from the first fluid 92 by the gas-liquid separator 41, 42 is fed to the xylene synthesis reactor 12.

In each of the systems 100 for producing aromatic hydrocarbons 97 according to items 2 and 3, the reaction to generate the aromatic hydrocarbons 97 from CO₂ and H₂ is divided into two stages, i.e., the methanol synthesis reaction performed by the methanol synthesis reactor 11 and the xylene synthesis reaction performed by the xylene synthesis reactor 12. Accordingly, suitable reaction conditions can be realized for each of the methanol synthesis reaction and the xylene synthesis reaction. Further, unreacted CO₂ and H₂ can be separated and returned to the methanol synthesis reactor 11 at a stage prior to their mixing with by-products such as aliphatic hydrocarbons 98, which are generated in the xylene synthesis reaction, and the aliphatic hydrocarbons 98 can be recovered in a subsequent step as valuable materials.

The system 100 for producing aromatic hydrocarbons 97 according to item 4 of the present disclosure is configured such that the system 100 for producing aromatic hydrocarbons 97 according to item 2 or 3 includes: first heat recovery equipment 32, which recovers thermal energy from the first fluid 92; second heat recovery equipment 34, which recovers thermal energy from a second fluid 96, which contains reaction products of the xylene synthesis reaction and which has been discharged from the xylene synthesis reactor 12; and a carbon dioxide feed source 46 including: a carbon dioxide absorbent 43, which has reversibly absorbed carbon dioxide; and an absorbent heater 53, which heats the carbon dioxide absorbent 43 with at least one of the thermal energy recovered from the first fluid 92 or the thermal energy recovered from the second fluid 96, wherein the carbon dioxide absorbent 43 desorbs the carbon dioxide when heated, and the desorbed carbon dioxide is fed from the carbon dioxide feed source 46 to the raw material gas line 21.

In a conventional system for producing aromatic hydrocarbons, in a case where CO₂ stored in a carbon dioxide absorbent is used as a raw material, for the procurement of the raw material gas, in particular, for the desorption of CO₂ from the carbon dioxide absorbent, the system is externally fed with a significantly large amount of energy, which results in a problem of high cost. On the other hand, in the system 100 for producing aromatic hydrocarbons 97 according to the present disclosure, heat generated in the process of producing the aromatic hydrocarbons 97 is effectively utilized to feed CO₂ to be contained in the raw material gas 91, i.e., effectively utilized for the procurement of the raw material gas 91. As a result, energy that is fed from the outside of the system for the procurement of the raw material gas 91 can be reduced, which makes it possible to reduce the cost for the procurement of the raw material gas 91. Further, since the energy is recycled within the system, energy loss is reduced, and consequently CO₂ emission is reduced.

The system 100 for producing aromatic hydrocarbons 97 according to item 5 of the present disclosure is configured such that, in the system 100 for producing aromatic hydrocarbons 97 according to item 4: the first heat recovery equipment 32 includes a first heat transfer medium passage 360, through which a first heat transfer medium 36 flows, the first heat transfer medium 36 recovering the thermal energy of the first fluid 92 by exchanging heat with the first fluid 92; the system 100 includes a raw material heater 31, which heats the raw material gas 91 by heat exchange between the raw material gas 91 and the first heat transfer medium 36; and the first heat transfer medium 36 is fed to the absorbent heater 53 through the first raw material heater 31.

In the production system 100 configured as described above, the thermal energy recovered from the first fluid 92 is utilized to heat the raw material gas 91, and then utilized to heat the carbon dioxide absorbent 43. The CO₂ desorption temperature of the carbon dioxide absorbent 43 is sufficiently lower than a suitable temperature of the raw material gas 91 for the methanol synthesis reaction. Therefore, even the thermal energy that has already been utilized to heat the raw material gas 91 can still be utilized for CO₂ desorption, and thus effective utilization of the thermal energy of the first fluid 92 is made possible.

The system 100 for producing aromatic hydrocarbons 97 according to item 6 of the present disclosure is configured such that, in the system 100 for producing aromatic hydrocarbons 97 according to item 4 or 5: the second heat recovery equipment 34 includes a second heat transfer medium passage 370, through which a second heat transfer medium 37 flows, the second heat transfer medium 37 recovering the thermal energy of the second fluid 96 by exchanging heat with the second fluid 96; the system 100 includes a methanol heater 33, which is located on the methanol feed line 23 and which heats the methanol 94 by heat exchange between the methanol 94 and the second heat transfer medium 37; and the second heat transfer medium 37 is fed to the absorbent heater 53 through the methanol heater 33.

In the above-described production system 100, the thermal energy recovered from the second fluid 96 is utilized to heat the methanol 94, and then utilized to heat the carbon dioxide absorbent 43. The CO₂ desorption temperature of the carbon dioxide absorbent 43 is sufficiently lower than a suitable temperature of the methanol 94 for the xylene synthesis reaction. Therefore, even the thermal energy that has already been utilized to heat the raw material gas 91 can still be utilized for CO₂ desorption, and thus effective utilization of the thermal energy of the second fluid 96 is made possible.

The system 100 for producing aromatic hydrocarbons 97 according to item 7 of the present disclosure is configured such that the system 100 for producing aromatic hydrocarbons 97 according to item 6 includes a second raw material heater 31, which is located on the raw material gas line 21 and which heats the raw material gas 91 by heat exchange between the raw material gas 91 and the second heat transfer medium 37, wherein the second heat transfer medium 37 is fed to the absorbent heater 53 through the methanol heater 33 and the raw material heater 31.

In the production system 100 configured as described above, the thermal energy recovered from the second fluid 96 is utilized to heat the methanol 94 and the raw material gas 91, and then utilized to heat the carbon dioxide absorbent 43. The CO₂ desorption temperature of the carbon dioxide absorbent 43 is sufficiently lower than a suitable temperature of the raw material gas 91 for the methanol synthesis reaction. Therefore, even the thermal energy that has already been utilized to heat the methanol 94 and the raw material gas 91 can still be utilized for CO₂ desorption, and thus effective utilization of the thermal energy of the second fluid 96 is made possible.

The system 100 for producing aromatic hydrocarbons 97 according to item 8 of the present disclosure is configured such that, in the system 100 for producing aromatic hydrocarbons 97 according to any one of items 4 to 7: the second heat recovery equipment 34 includes a second heat transfer medium passage 370, through which a second heat transfer medium 37 flows, the second heat transfer medium 37 recovering the thermal energy of the second fluid 96 by exchanging heat with the second fluid 96; the system 100 includes a water vapor heater 35, which is located on the water vapor feed line 24 and which heats the water vapor 95 by heat exchange between the water vapor 95 and the second heat transfer medium 37; and the second heat transfer medium 37 is fed to the absorbent heater 53 through the water vapor heater 35.

In the above-described production system 100, the energy recovered from the second fluid 96 is utilized to heat the water vapor 95, and then utilized to heat the carbon dioxide absorbent 43. The CO₂ desorption temperature of the carbon dioxide absorbent 43 is sufficiently lower than a suitable temperature of the water vapor 95 for the xylene synthesis reaction. Therefore, even the thermal energy that has already been utilized to heat the water vapor 95 can still be utilized for CO₂ desorption, and thus effective utilization of the thermal energy of the second fluid 96 is made possible.

The system 100 for producing aromatic hydrocarbons 97 according to item 9 of the present disclosure is configured such that the system 100 for producing aromatic hydrocarbons 97 according to item 8 includes a second raw material heater 31, which is located on the raw material gas line 21 and which heats the raw material gas 91 by heat exchange between the raw material gas 91 and the second heat transfer medium 37, wherein the second heat transfer medium 37 is fed to the absorbent heater 53 through the water vapor heater 35 and the raw material heater 31.

In the above-described production system 100, the thermal energy recovered from the second fluid 96 is utilized to heat the water vapor 95 and the raw material gas 91, and then utilized to heat the carbon dioxide absorbent 43. The CO₂ desorption temperature of the carbon dioxide absorbent 43 is sufficiently lower than a suitable temperature of the raw material gas 91 for the methanol synthesis reaction. Therefore, even the thermal energy that has already been utilized to heat the water vapor 95 and the raw material gas 91 can still be utilized for CO₂ desorption, and thus effective utilization of the thermal energy of the second fluid 96 is made possible.

A method of producing aromatic hydrocarbons 97 according to item 10 of the present disclosure includes: feeding methanol 94 to a xylene synthesis reactor 12 filled with a xylene synthesis catalyst 120; feeding water vapor 95, 99a to the xylene synthesis reactor 12; and synthesizing, by the xylene synthesis reactor 12, aromatic hydrocarbons 97 from the methanol 94 by a xylene synthesis reaction in the presence of the water vapor 95, 99a.

In the above-described method of producing aromatic hydrocarbons 97, the methanol 94 is converted into the aromatic hydrocarbons 97 by the xylene synthesis catalyst 120 in the presence of the water vapor 99a, 95. It has been found that by bringing the methanol 94 into contact with the xylene synthesis catalyst 120 in the presence of the water vapor 99a, 95, the aromatic hydrocarbons 97 can be generated while reducing the deactivation rate of the catalyst 120. By reducing the deactivation rate of the xylene synthesis catalyst 120, the service life of the xylene synthesis catalyst 120 can be extended, and consequently the frequency of the replacement of the xylene synthesis catalyst 120 can be reduced.

The method of producing aromatic hydrocarbons 97 according to item 11 of the present disclosure is such that the method of producing aromatic hydrocarbons 97 according to item 10 includes: feeding raw material gas 91 containing hydrogen and carbon dioxide to a methanol synthesis reactor 11; generating, by the methanol synthesis reactor 11, a first fluid 92 containing the methanol 94 and water 99 from the raw material gas 91 by a methanol synthesis reaction; separating the methanol 94 and the water 99 from the first fluid 92; and feeding the methanol 94 and the water 99 separated from the first fluid 92 to the xylene synthesis reactor 12 as the methanol 94 and the water vapor 99a.

In the above-described production method, water generated from the methanol synthesis reaction can be effectively utilized for the xylene synthesis reaction.

The method of producing aromatic hydrocarbons 97 according to item 12 of the present disclosure is such that the method of producing aromatic hydrocarbons 97 according to item 10 includes: feeding raw material gas 91 containing hydrogen and carbon dioxide to a methanol synthesis reactor 11; generating, by the methanol synthesis reactor 11, a first fluid 92 containing the methanol 94 from the raw material gas 91 by a methanol synthesis reaction; separating the methanol 94 from the first fluid 92; and feeding the methanol 94 separated from the first fluid 92 to the xylene synthesis reactor 12.

In each of the methods of producing aromatic hydrocarbons 97 according to items 11 and 12, the reaction to generate the aromatic hydrocarbons 97 from CO₂ and H₂ is divided into two stages, i.e., the methanol synthesis reaction performed by the methanol synthesis reactor 11 and the xylene synthesis reaction performed by the xylene synthesis reactor 12. Accordingly, suitable reaction conditions can be realized for each of the methanol synthesis reaction and the xylene synthesis reaction. Further, unreacted CO₂ and H₂ can be separated and returned to the methanol synthesis reactor 11 at a stage prior to their mixing with by-products such as aliphatic hydrocarbons 98, which are generated in the xylene synthesis reaction, and the aliphatic hydrocarbons 98 can be recovered in a subsequent step as valuable materials.

The method of producing aromatic hydrocarbons 97 according to item 13 of the present disclosure is such that the method of producing aromatic hydrocarbons 97 according to item 11 or 12 includes: recovering thermal energy from the first fluid 92; recovering thermal energy from a second fluid 96 containing the aromatic hydrocarbons 97 synthesized by the xylene synthesis reactor 12 by the xylene synthesis reaction; separating the aromatic hydrocarbons 97 from the second fluid 96; and heating a carbon dioxide absorbent 43 with at least one of the thermal energy recovered from the first fluid 92 or the thermal energy recovered from the second fluid 96 to desorb carbon dioxide from the carbon dioxide absorbent 43, and using the desorbed carbon dioxide as a material for the raw material gas 91.

In the above-described method of producing aromatic hydrocarbons 97, heat generated in the process of producing the aromatic hydrocarbons 97 is effectively utilized to feed CO₂ to be contained in the raw material gas 91, i.e., effectively utilized for the procurement of the raw material gas 91. As a result, energy that is fed from the outside of the system for the procurement of the raw material gas 91 can be reduced, which makes it possible to reduce the cost for the procurement of the raw material gas 91. Further, since the energy is recycled within the system, energy loss is reduced, and consequently CO₂ emission is reduced.

The present disclosure has been discussed as above for the purpose of presenting examples and explanations, and the above discussion has no intention to limit the present disclosure to the modes disclosed herein. For example, in the above detailed description, various features of the present disclosure are grouped together in several embodiments for the purpose of streamlining the present disclosure. However, some of the features therein may be combined. Further, the features included in the present disclosure may be combined with alternative embodiments, alternative configurations, or alternatively modes, other than those described above.

### Reference Signs List

11: methanol synthesis reactor
12: xylene synthesis reactor
21: raw material gas line
23: methanol feed line
24: water vapor feed line
31: raw material heater
32: first heat recovery equipment
33: methanol heater
34: second heat recovery equipment
35: water vapor heater
36: first heat transfer medium
37: second heat transfer medium
41: first gas-liquid separator
42: second gas-liquid separator
43: carbon dioxide absorbent
46: carbon dioxide feed source
53: absorbent heater
91: raw material gas
92: first fluid
94: methanol
95: water vapor
96: second fluid
97: aromatic hydrocarbon
360: first heat transfer medium passage
370: second heat transfer medium passage
100: aromatic hydrocarbon production system
120: xylene synthesis catalyst

## Claims

1. A system for producing aromatic hydrocarbons, the system comprising:
a xylene synthesis reactor that is filled with a xylene synthesis catalyst and that synthesizes aromatic hydrocarbons from methanol by a xylene synthesis reaction in the presence of water vapor;
a methanol feed line through which the methanol is fed to the xylene synthesis reactor; and
a water vapor feed line through which the water vapor is fed to the xylene synthesis reactor.

2. The system for producing aromatic hydrocarbons according to claim 1, comprising:
a methanol synthesis reactor that synthesizes the methanol from raw material gas containing hydrogen and carbon dioxide by a methanol synthesis reaction accompanied by water vapor generation;
a raw material gas line through which the raw material gas is fed to the methanol synthesis reactor; and
a gas-liquid separator that separates the methanol and water from a first fluid that contains reaction products of the methanol synthesis reaction and that has been discharged from the methanol synthesis reactor, wherein:
through the methanol feed line, the methanol and the water separated from the first fluid by the gas-liquid separator are fed to the xylene synthesis reactor as the methanol and the water vapor; and
the methanol feed line doubles as the water vapor feed line.

3. The system for producing aromatic hydrocarbons according to claim 1, comprising:
a methanol synthesis reactor that synthesizes the methanol from raw material gas containing hydrogen and carbon dioxide by a methanol synthesis reaction;
a raw material gas line through which the raw material gas is fed to the methanol synthesis reactor;
a gas-liquid separator that separates the methanol from a first fluid that contains reaction products of the methanol synthesis reaction and that has been discharged from the methanol synthesis reactor; and
the methanol feed line through which the methanol separated from the first fluid by the gas-liquid separator is fed to the xylene synthesis reactor.

4. The system for producing aromatic hydrocarbons according to claim 2 or 3, comprising:
first heat recovery equipment that recovers thermal energy from the first fluid;
second heat recovery equipment that recovers thermal energy from a second fluid that contains reaction products of the xylene synthesis reaction and that has been discharged from the xylene synthesis reactor; and
a carbon dioxide feed source including:
a carbon dioxide absorbent that has reversibly absorbed carbon dioxide; and
an absorbent heater that heats the carbon dioxide absorbent with at least one of the thermal energy recovered from the first fluid or the thermal energy recovered from the second fluid, wherein
the carbon dioxide absorbent desorbs the carbon dioxide when heated, and the desorbed carbon dioxide is fed from the carbon dioxide feed source to the raw material gas line.

5. The system for producing aromatic hydrocarbons according to claim 4, wherein:
the first heat recovery equipment includes a first heat transfer medium passage through which a first heat transfer medium flows, the first heat transfer medium recovering the thermal energy of the first fluid by exchanging heat with the first fluid;
the system comprises a first raw material heater that heats the raw material gas by heat exchange between the raw material gas and the first heat transfer medium; and
the first heat transfer medium is fed to the absorbent heater through the first raw material heater.

6. The system for producing aromatic hydrocarbons according to claim 4, wherein:
the second heat recovery equipment includes a second heat transfer medium passage through which a second heat transfer medium flows, the second heat transfer medium recovering the thermal energy of the second fluid by exchanging heat with the second fluid;
the system comprises a methanol heater that is located on the methanol feed line and that heats the methanol by heat exchange between the methanol and the second heat transfer medium; and
the second heat transfer medium is fed to the absorbent heater through the methanol heater.

7. The system for producing aromatic hydrocarbons according to claim 6, comprising a second raw material heater that is located on the raw material gas line and that heats the raw material gas by heat exchange between the raw material gas and the second heat transfer medium, wherein
the second heat transfer medium is fed to the absorbent heater through the methanol heater and the second raw material heater.

8. The system for producing aromatic hydrocarbons according to claim 4, wherein:
the second heat recovery equipment includes a second heat transfer medium passage through which a second heat transfer medium flows, the second heat transfer medium recovering the thermal energy of the second fluid by exchanging heat with the second fluid;
the system comprises a water vapor heater that is located on the water vapor feed line and that heats the water vapor by heat exchange between the water vapor and the second heat transfer medium; and
the second heat transfer medium is fed to the absorbent heater through the water vapor heater.

9. The system for producing aromatic hydrocarbons according to claim 8, comprising a second raw material heater that is located on the raw material gas line and that heats the raw material gas by heat exchange between the raw material gas and the second heat transfer medium, wherein
the second heat transfer medium is fed to the absorbent heater through the water vapor heater and the second raw material heater.

10. A method of producing aromatic hydrocarbons, the method comprising:
feeding methanol to a xylene synthesis reactor filled with a xylene synthesis catalyst;
feeding water vapor to the xylene synthesis reactor; and
synthesizing, by the xylene synthesis reactor, aromatic hydrocarbons from the methanol by a xylene synthesis reaction in the presence of the water vapor.

11. The method of producing aromatic hydrocarbons according to claim 10, comprising:
feeding raw material gas containing hydrogen and carbon dioxide to a methanol synthesis reactor;
generating, by the methanol synthesis reactor, a first fluid containing the methanol and water from the raw material gas by a methanol synthesis reaction;
separating the methanol and the water from the first fluid; and
feeding the methanol and the water separated from the first fluid to the xylene synthesis reactor as the methanol and the water vapor.

12. The method of producing aromatic hydrocarbons according to claim 10, comprising:
feeding raw material gas containing hydrogen and carbon dioxide to a methanol synthesis reactor;
generating, by the methanol synthesis reactor, a first fluid containing the methanol from the raw material gas by a methanol synthesis reaction;
separating the methanol from the first fluid; and
feeding the methanol separated from the first fluid to the xylene synthesis reactor.

13. The method of producing aromatic hydrocarbons according to claim 11 or 12,
comprising:
recovering thermal energy from the first fluid;
recovering thermal energy from a second fluid containing the aromatic hydrocarbons synthesized by the xylene synthesis reactor by the xylene synthesis reaction;
separating the aromatic hydrocarbons from the second fluid; and
heating a carbon dioxide absorbent with at least one of the thermal energy recovered from the first fluid or the thermal energy recovered from the second fluid to desorb carbon dioxide from the carbon dioxide absorbent, and using the desorbed carbon dioxide as a material for the raw material gas.
